# FASCICULE DE BREVET EUROPEEN

(11) **EP 0 890 577 B1**
(45) Date de publication et mention de la délivrance du brevet: **06.11.2002**
(21) Numéro de dépôt: 98401499.3
(22) Date de dépôt: 18.06.1998
(51) Int. Cl.: C07J 41/00, A61K 9/127, A61K 7/00

(54) **Composition comprenant une dispersion aqueuse de vésicules lipidiques à base de carbamates à chaîne cholestéryle, utilisation notamment en cosmétique et nouveaux composés**
Auf Cholesterylcarbamaten basierende Zusammensetzung, die eine wässrige Dispersion von Lipidvesikulen enthält, kosmetische Verwendung und Verbindungen
Composition comprising an aqueous dispersion of lipid vesicules, based on cholesteryl carbamates, cosmetic use and compounds

(30) Priorité: 10.07.1997 FR 9708801
(43) Date de publication de la demande: 13.01.1999
(73) Titulaire: L'OREAL, 75008 Paris (FR)
(72) Inventeur: Philippe, Michel, 91320 Wissous (FR); Blaise, Christian, 94160 Saint Mande (FR); Simonnet, Jean-Thierry, 75011 Paris (FR)
(74) Mandataire: Dodin, Catherine

(56) Documents cités:
- WO-A-93/05162
- WO-A-96/18372
- US-A- 4 707 453
- US-A- 5 614 503

## Description

La présente invention concerne une composition comprenant une dispersion aqueuse de vésicules lipidiques à base de carbamate à chaîne cholestéryle et son utilisation en application topique.

Les dispersions de vésicules lipidiques sont bien connues dans le domaine cosmétique ou en dermopharmacie.
On sait que certains lipides amphiphiles possèdent la propriété de former des phases mésomorphes, dont l'état d'organisation est intermédiaire entre l'état cristallin et l'état liquide et que, parmi eux, certains sont susceptibles de gonfler en présence d'une solution aqueuse pour former une phase lamellaire puis, après agitation, former des vésicules ou sphérules dispersées dans une phase aqueuse. Ces vésicules sont formées par une membrane constituée par des feuillets sensiblement concentriques comportant une ou plusieurs couches multimoléculaires, de préférence bimoléculaires, encapsulant une phase aqueuse.
Parmi les lipides aptes à former des vésicules lipidiques, il est connu selon la demande WO-A-83/04412 des composés amides comprenant un groupement dérivé de sucres tels que le D-mannose, le D-galactose et le D-glucose.
Les vésicules susmentionnées peuvent être préparées par de nombreux procédés connus.
Selon un premier procédé, qui est par exemple décrit par BANGHAM et al. (J. Mol. Bio., 13, 1965- pages 238 à 262), on dissout la phase lipidique dans un solvant volatil, on forme un film mince de phase lipidique sur les parois d'un flacon par évaporation du solvant, on introduit la phase aqueuse à encapsuler sur le film lipidique et on agite le mélange mécaniquement jusqu'à obtention de la dispersion de vésicules à la taille désirée ; on obtient ainsi une dispersion aqueuse de vésicules encapsulant une phase aqueuse. Ce procédé nécessite l'emploi d'un solvant lors de la préparation de la phase lipidique.
Selon un second procédé dit "par cofusion des lipides", décrit par exemple dans FR-A-2315991, on prépare la phase lipidique par mélange du (des) lipide(s) amphiphile(s) et des éventuels additifs, à une température où le mélange est fondu, si le mélange n'est pas liquide à température ambiante ; on forme une phase lamellaire, par introduction de la phase aqueuse à encapsuler ; puis on disperse la phase lamellaire sous forme de vésicules, à l'aide d'un ultra-disperseur, d'un homogénéiseur ou d'ultrasons, dans une phase aqueuse de dispersion. Cet autre procédé nécessite de chauffer les lipides pour atteindre leur température de fusion.
Par ailleurs, pour obtenir des dispersions de vésicules lipidiques stables dans le temps, il est connu, notamment par la demande EP-A-582503 d'associer aux lipides un additif apte à améliorer les propriétés d'encapsulation de la membrane lipidique. Le cholestérol est l'additif le plus couramment utilisé dans ce but. La préparation de ces dispersions de vésicules nécessite donc une étape supplémentaire pour incorporer l'additif dans le mélange de lipides.

Ainsi, ces procédés nécessitent de nombreuse étapes pour préparer les dispersions de vésicules lipidiques et ces étapes rendent les procédés assez complexes à mettre en oeuvre.
En outre, des dispersions aqueuses de vésicules ne sont pas toujours stables dans le temps selon les lipides présents dans la phase lipidique des vésicules. Cette instabilité peut être due, par exemple, à la recristallisation de certains lipides de la membrane des vésicules, ou à l'oxydation dans le temps des lipides, ou bien encore à la désintégration de la structure de la membrane lipidique.
Il est souhaitable également que les vésicules lipidiques possèdent de bonnes propriétés d'encapsulation pour permettre de véhiculer des ingrédients actifs selon l'application recherchée. On recherche donc à obtenir des vésicules lipidiques ayant une bonne perméabilité.

La présente invention a pour but de proposer des dispersions aqueuses de vésicules lipidiques qui peuvent être préparées selon un procédé plus simple que ceux de l'art antérieur. L'invention a aussi pour but de proposer des dispersions aqueuses de vésicules présentant de bonnes propriétés d'encapsulation et de stabilité.

La Demanderesse a découvert que ces buts sont atteints en utilisant comme lipide des carbamates particuliers, à chaîne cholestéryle, pour la préparation de dispersions aqueuses de vésicules lipidiques.
On a en effet constaté que les dispersions aqueuses de vésicules peuvent être préparées par simple dispersion de ces lipides dans l'eau. La dispersion aqueuse selon l'invention est ainsi préparée selon un procédé plus simple que les procédés connus de l'art antérieur.
De plus, les dispersions aqueuses de vésicules de l'invention présentent de bonnes propriétés d'encapsulation : les vésicules présentent un faible taux de fuite, et sont parfaitement stables dans l'eau. En outre, les carbamates à chaînes cholestéryle ont un taux de gonflement élevé et permettent donc la formation de vésicules présentant un bon taux d'encapsulation.
Les dispersions aqueuses de vésicules de l'invention peuvent être, par conséquent, conservées et être utilisées pour la préparation de compositions à usage topique.

L'invention a donc pour objet une composition comprenant une dispersion aqueuse de vésicules comprenant une membrane lipidique, caractérisée par le fait que ladite membrane lipidique comprend au moins un carbamate de formule (I) suivante: 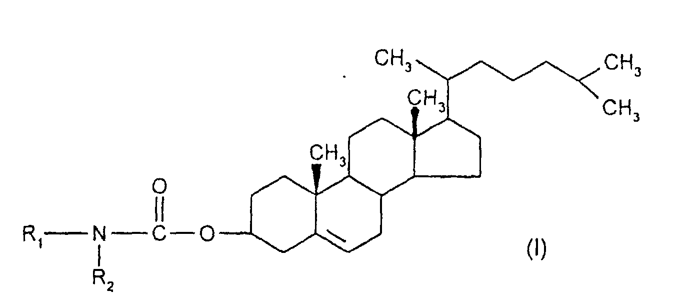 dans laquelle :
- R₁ désigne un atome d'hydrogène ou un radical alkyle, linéaire ou ramifié, saturé ou insaturé, éventuellement hydroxylé, ayant de 1 à 6 atomes de carbone,
- R₂ désigne un radical alkyle, linéaire ou ramifié, saturé ou insaturé, éventuellement hydroxylé, ayant de 3 à 6 atomes de carbone,
sous réserve que le groupement (R₁)(R₂)N- comporte au moins deux groupements hydroxyle.

L'invention porte également sur un procédé de traitement non thérapeutique de la peau et/ou du cuir chevelu, caractérisé par le fait qu'on applique sur la peau et/ou le cuir chevelu, une composition telle que définie précédemment.
L'invention a également pour objet l'utilisation de vésicules comprenant une membrane lipidique qui comprend au moins un carbamate de formule (I), pour disperser ou aider à la mise en dispersion, d'un liquide non miscible à l'eau, dans une phase aqueuse.
Un autre objet de l'invention est l'utilisation de carbamate de formule (I) pour la préparation de vésicules lipidiques.

Parmi les composés de formule (I), on peut notamment citer :
- le N-cholestéryloxycarbonyl-N-méthyl-D-glucamine,
- le N-cholestéryloxycarbonyl-D-glucamine,
- le N-cholestéryloxycarbonyl-1,1-di(hydroxyméthyl)-éthylamine,
- le N-cholestéryloxycarbonyl-2,3-dihydroxy-propylamine.

Les composés de formule (I) peuvent être préparés selon des procédés connus de l'homme du métier.
Par exemple, ces composés peuvent être obtenus par réaction d'un amino-alcool de formule R₁-NH-R₂ avec le chloroformiate de cholestéryle, en présence de solvant tel que le N,N-diméthyl-acétamide.
Certains des composés de formule (I) sont notamment décrits dans les demandes WO-A-91/13080, WO-A-92/03464, EP-A-430078, ainsi que dans les publications Lange W., Amundson M., Journal of pharmaceutical sciences, vol 51, n° 11, (1962), p 1102-1106 et Reed M. et al, Journal of medicinal chemistry, vol 38, n° 22, (1995), p 4587-4596.

Les vésicules lipidiques selon l'invention peuvent encapsuler une phase aqueuse (vésicules lipidiques à coeur aqueux) ou une phase huileuse (vésicules lipidiques à coeur huileux).

Les vésicules lipidiques à coeur aqueux conformes à l'invention comprennent, de préférence, une membrane lipidique formée d'au moins un carbamate de formule (I) et d'au moins un lipide amphiphile ionique. La membrane lipidique peut comprendre en outre au moins un lipide amphiphile non-ionique associé audit lipide amphiphile ionique. Ces vésicules lipidiques à coeur aqueux peuvent être préparées par tout procédé connu de fabrication des vésicules lipidiques amphiphiles et plus particulièrement selon les procédés décrits dans la demande EP-A-504437.
De préférence, le rapport en poids entre la quantité de carbamate de formule (I) et la quantité de lipide amphiphile est compris entre 30/1 et 50/25, et le rapport en poids entre la quantité de membrane lipidique et la quantité de phase aqueuse de la dispersion est compris entre 1/1000 et 300/1000.
Les vésicules à coeur aqueux conformes à l'invention ont avantageusement un diamètre moyen allant de 10 à 1000 nm.
Les vésicules à coeur aqueux selon l'invention sont présents dans la composition dans des proportions allant, de préférence, de 0,5 à 15 % en poids par rapport au poids total de la composition.
On peut, de façon connue, incorporer dans la membrane lipidique des vésicules à coeur aqueux, au moins un additif qui a pour fonction principale de diminuer la perméabilité des vésicules, de prévenir leur floculation et leur fusion et d'augmenter le taux d'encapsulation.
Selon un mode préférentiel de l'invention, on peut ajouter à ladite membrane lipidique au moins un additif choisi, de préférence, dans le groupe formé par :
- les stérols et notamment les phytostérols et le cholestérol,
- les alcools et diols à longue chaîne,
- les amines à longue chaîne et leurs dérivés ammonium quaternaire.
Ces additifs peuvent éventuellement avoir une activité cosmétique et/ou dermopharmaceutique. C'est, par exemple, le cas du cholestérol.

Les vésicules à coeur huileux, utilisés selon la présente invention, se présentent de préférence sous la forme de globules huileux en dispersion unitairement enrobés d'une couche monolamellaire ou oligolamellaire (2 à 5 lamelles lipidiques) obtenue à partir d'au moins un carbamate de formule (I) et d'un tensioactif lipophile et/ou d'un tensioactif hydrophile. Avantageusement, la couche monolamellaire ou oligolamellaire peut comprendre aussi soit un lipide amphiphile ionique soit un acide gras associé à un agent basique dissous dans la phase aqueuse de la dispersion.

Les vésicules lipidiques à coeur huileux conformes à l'invention peuvent être préparés selon le procédé de fabrication décrit dans les demandes FR-A-2709666 et FR-A-2725369. Ce procédé consiste dans une première étape à mélanger sous agitation la phase grasse contenant le (ou les) ester(s) d'acide gras d'αbutylglucoside, le tensioactif hydrophile ou lipophile, le lipide amphiphile ionique ou l'acide gras et la phase aqueuse et dans une deuxième étape à soumettre le mélange obtenu à une homogénéisation basée sur le principe de la cavitation. Cette homogénéisation est obtenue soit à l'aide de hautes pressions comprises entre 200 et 1500 bars, soit à l'aide d'ultrasons, soit à l'aide d'homogénéisateurs équipés d'une tête rotor-stator.
Les vésicules lipidiques à coeur huileux tels que décrits ci-dessus ont de préférence une taille moyenne allant de 10 à 500 nanomètres et de préférence de 10 à 200 nanomètres.
Les vésicules à coeur huileux selon l'invention sont présents dans la composition dans des proportions allant, de préférence, de 5 à 50 % en poids par rapport au poids total de la composition.

Les lipides amphiphiles ioniques associés aux carbamates de formule (I) peuvent être choisis dans le groupe formé par :
1) les lipides anioniques neutralisés, ces lipides anioniques étant de préférence, choisis parmi :
   - les sels alcalins du dicétylphosphate, et du dimyristylphosphate en particulier les sels de Na et K ;
   - les sels alcalins du cholestérol-sulfate, en particulier le sel de Na ;
   - les sels alcalins du cholestérol-phosphate, en particulier le sel de Na ;
   - les sels de lipoaminoacides tels que les acylglutamates mono- et disodique ;
   - le sel de sodium de l'acide phosphatidique ;
2) les lipides amphotères, ces lipides amphotères étant, de préférence, des phospholipides, en particulier la phosphatidyléthanolamine de soja pure ;
3) les dérivés alkylsulfoniques, ces dérivés étant de préférence, les composés de formule : 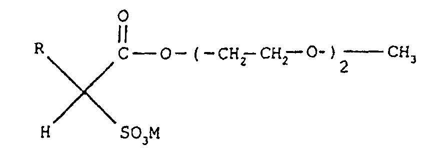 formule dans laquelle R représente des radicaux en C₁₂-C₂₂, en particulier les radicaux C₁₆H₃₃ et C₁₈H₃₇ pris en mélange ou séparément et M est un métal alcalin, de préférence le sodium.

Les lipides non-ioniques amphiphiles utilisés pour la préparation des vésicules à coeur aqueux sont choisis de préférence dans le groupe formé par :
1) les éthers de polyglycérol, linéaires ou ramifiés, de formule (II) :

   R¹⁰O-[-C₃H₅-(OH)O-]ₙ-H

   dans laquelle :
   . -C₃H₅(OH)O est représenté par les structures suivantes prises en mélange ou séparément:

      -CH₂CHOHCH₂O ; 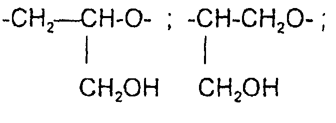
   . n est une valeur statistique moyenne comprise entre 2 et 6 ;
   . R¹⁰ représente :
      (a) une chaîne aliphatique, linéaire ou ramifiée, contenant de 12 à 18 atomes de carbone ;
      (b) un reste R¹¹CO, où R¹¹ est un radical aliphatique, linéaire ou ramifié, en C₁₁-C₁₇ ;
      (c) un reste R¹²-[-OC₂H₃(R¹³)-]-, où :
   . R¹² peut prendre la signification (a) ou (b) donnée pour R¹⁰ ;
   . OC₂H₃(R¹³)- est représenté par les structures suivantes, prises en mélange ou séparément : 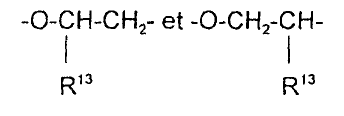 où R¹³ prend la signification (a) donnée pour R¹⁰;
(2) les alcools gras polyoxyéthylénés, les stérols polyoxyéthylénés ;
(3) les esters de polyols éventuellement polyoxyéthylénés ;
(4) les glycolipides naturels ou synthétiques ; et
(5) le stéarate de polyglycérol oxyéthyléné.

Les agents tensioactifs lipophiles et les agents tensioactifs hydrophiles utilisés pour la préparation des vésicules à coeur huileux comportent de préférence au moins une chaîne grasse saturée ayant plus de 12 atomes de carbone environ. Plus préférentiellement encore, cette chaîne grasse contient de 16 à 22 atomes de carbone.

Selon un autre mode de réalisation préférentiel de l'invention, l'agent tensioactif lipophile présente un HLB compris entre environ 2 et environ 5. Comme cela est bien connu, on entend par HLB (Hydrophilic-Lipophilic Balance) l'équilibre entre la dimension et la force du groupe hydrophile et la dimension et la force du groupe lipophile de l'agent tensioactif.

Des exemples de tels agents tensioactifs lipophiles sont le distéarate de sucrose, le distéarate de diglycéryle, le tristéarate de tétraglycéryle, le décastéarate de décaglycéryle, le monostéarate de diglycéryle, le tristéarate d'hexaglycéryle, le pentastéarate de décaglycéryle, le monostéarate de sorbitane, le tristéarate de sorbitane, le monostéarate de diéthylène glycol, l'ester de glycérol et d'acides palmitique et stéarique, le monostéarate polyoxyéthyléné 2 OE (comportant 2 motifs oxyéthylène), le mono et dibéhénate de glycéryle, le tétrastéarate de pentaérythritol.
L'agent tensioactif hydrophile présente de préférence un HLB compris entre environ 8 et environ 12. On peut citer comme exemples de tels tensioactifs hydrophiles, les composés suivants le monostéarate de sorbitane polyoxyéthyléné 4 OE, le tristéarate de sorbitane polyoxyéthyléné 20 OE, le monostéarate polyoxyéthyléné 8 OE, le monostéarate d'hexaglycéryle, le monostéarate polyoxyéthyléné 10 OE, le distéarate polyoxyéthyléné 12 OE et le distéarate de méthylglucose polyoxyéthyléné 20 OE.
Selon la valeur du HLB du ou des carbamates de formule (I) utilisés pour la préparation des vésicules à coeur huileux, on utilisera soit un tensioactif lipophile tel que défini ci-dessus, soit un tensioactif hydrophile tel que défini ci-dessus, soit l'association d'un tensioactif lipophile et d'un tensioactif hydrophile.
L'acide gras mis en oeuvre pour la préparation des vésicules à coeur huileux de l'invention est, de préférence, un acide gras saturé ayant de 16 à 22 atomes de carbone. On peut citer par exemple l'acide palmitique, l'acide stéarique, l'acide arachidique et l'acide béhénique.
L'agent basique contenu dans la phase aqueuse de la dispersion de vésicules à coeur huileux selon l'invention, est destiné à la neutralisation de l'acide gras présent dans la phase huileuse. Il doit donc être présent en une quantité au moins égale à celle nécessaire à la neutralisation de la totalité de l'acide gras. Il peut être choisi par exemple parmi la soude, la triéthanolamine, la lysine ou bien encore l'arginine.

Les vésicules des compositions selon l'invention peuvent contenir, de façon connue, un ou plusieurs composé(s) actif(s) ayant une activité cosmétique et/ou dermopharmaceutique, qui, selon leurs caractéristiques de solubilité, peuvent avoir différentes localisations. Si les actifs sont hydrosolubles, on les introduit, de préférence, dans la phase aqueuse encapsulée des vésicules à coeur aqueux. Si les actifs sont liposolubles, on les introduit, de préférence, dans la phase lipidique constituant la membrane ou bien dans la phase huileuse encapsulée des vésicules à coeur huileux.
Si les actifs sont amphiphiles, ils se répartissent entre la membrane lipidique et la phase aqueuse encapsulée des vésicules à coeur aqueux ou bien entre la membrane lipidique et la phase aqueuse de la dispersion des vésicules à coeur huileux, avec un coefficient de partage qui varie selon la nature de l'actif amphiphile et les compositions respectives des différentes phases en contact avec l'actif.
Les actifs hydrosolubles sont, par exemple, la glycérine, le sorbitol, l'érythrulose et les antibiotiques. Les actifs liposolubles ou partiellement liposolubles (amphiphiles) sont choisis parmi ceux qui n'augmentent pas de façon significative la perméabilité des vésicules, ne provoquent pas leur floculation et leur fusion et ne diminuent pas le taux d'encapsulation. On utilise avantageusement des actifs liposolubles qui constituent également des additifs.

Une forme particulièrement préférée de vésicules lipidiques à coeur aqueux consiste en des vésicules comprenant une membrane lipidique obtenue à partir d'un mélange de composé de formule (I) tel que défini précédemment et de sel monosodique de glutamate de suif hydrogéné, notamment celui commercialisé sous la dénomination AMISOFT HS-11® par la société AJINOMOTO

La phase aqueuse de la dispersion conforme à l'invention peut contenir en outre un liquide non miscible à l'eau dispersé dans la phase aqueuse par les vésicules lipidiques.
Dans ce cas, les compositions conformes à la présente invention sont plus particulièrement des dispersions huile-dans-eau dans lesquelles les vésicules lipidiques agissent comme dispersant de l'huile dans la phase continue aqueuse.
Le liquide non miscible à l'eau, présent sous forme de dispersion dans la phase aqueuse, peut notamment être choisi dans le groupe formé par :
- les huiles animales ou végétales formées par des esters d'acides gras et de polyols, en particulier les triglycérides liquides, par exemple les huiles de tournesol, de maïs, de soja, de courge, de pépins de raisin, de jojoba, de sésame, de noisette, les huiles de poisson, le tricaprocaprylate de glycérol, ou les huiles végétales ou animales de formule R₉COOR₁₀, formule dans laquelle R₉ représente le reste d'un acide gras supérieur comportant de 7 à 19 atomes de carbone et R₁₀ représente une chaîne hydrocarbonée ramifiée contenant de 3 à 20 atomes de carbone, par exemple l'huile de Purcellin ;
- les huiles essentielles naturelles ou synthétiques telles que, par exemple, les huiles d'eucalyptus, de lavandin, de lavande, de vétiver, de litsea cubeba, de citron, de santal, de romarin, de camomille, de sarriette, de noix de muscade, de cannelle, d'hysope, de carvi, d'orange, de géraniol, de cade et de bergamote ;
- les hydrocarbures, tels que l'hexadécane et l'huile de paraffine ;
- les carbures halogénés, notamment des fluorocarbures tels que des fluoroamines par exemple la perfluorotributylamine, des hydrocarbures fluorés, par exemple le perfluorodécahydronaphtalène, des fluoroesters et des fluoroéthers ;
- les silicones, par exemple les polysiloxanes, les polydiméthylsiloxanes et les fluorosilicones ;
- les esters d'acide minéral et d'alcool ; et
- les éthers et les polyéthers.

La phase aqueuse de la dispersion peut également contenir des actifs cosmétiques et/ou dermopharmaceutiques, hydrosolubles. Le liquide non miscible à l'eau peut éventuellement contenir un actif liposoluble.
La phase aqueuse de la dispersion peut aussi contenir des adjuvants n'ayant ni activité cosmétique ni activité dermopharmaceutique propre, mais utilisés pour la formulation de la dispersion sous forme de lotion, crème, lait ou sérum. Ces adjuvants sont, en particulier, pris dans le groupe formé par les gélifiants, les conservateurs, les colorants, les opacifiants, les parfums, les pigments, les filtres solaires et les poudres à usage cosmétique. Parmi les gélifiants utilisables, on peut citer les dérivés d'algues tels que le satiagum, des gommes naturelles, telles que l'adragante, et des polymères synthétiques, en particulier les mélanges d'acides polycarboxyvinyliques commercialisés sous la dénomination "CARBOPOL" par la société GOODRICH.

Les compositions selon l'invention trouvent en particulier une application en tant que composition à usage topique.
Notamment les compositions telles que définies précédemment peuvent être utilisées comme, ou pour la préparation de, produits pour le soin et/ou le maquillage du visage, du corps et/ou du cuir chevelu. Ces produits peuvent se présenter sous la forme de dispersion plus ou moins épaissie, de gel, de crème, de lait ou de sérum.

L'invention concerne aussi les nouveaux composés de formule (I') suivante : 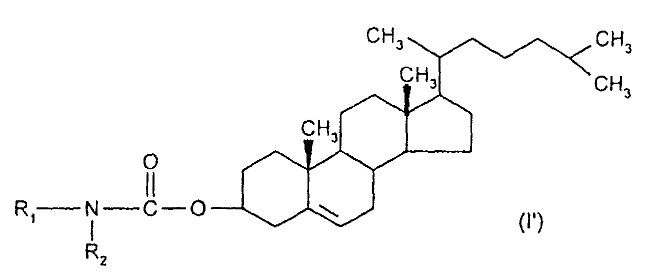 dans laquelle R₁ et R₂ ont la même signification que celle indiquée précédemment pour les composés de formule (I), à l'exception :
- du N-cholestéryloxycarbonyl-N-méthyl-D-glucamine (R₁ est méthyl et R₂ est le radical -CH₂-(CH OH)₄-CH₂OH et
- des composés pour lesquels :
- soit R₁ désigne un atome d'hydrogène et R₂ est un groupement choisi parmi :
   -CH(OH)-CH₂-CH₂-OH; -CH₂-CH(OH)-CH₂-OH ; -CH(CH₂OH)-CH₂-OH ; - CH₂-CH(OH)-CH₂-CH₂OH
- soit R₁ désigne un radical alkyle choisi dans le groupe formé par les radicaux méthyle, éthyle, n-propyl, isopropyl, n-butyl et t-butyl et R₂ désigne un groupement choisi parmi :
   -CH₂-CH(OH)-CH₂-OH ; -CH(CH₂OH)-CH₂-OH
- soit R₁ et R₂ désignent un radical -CH₂-CH₂OH.

Les exemples ci-après, donnés à titre purement illustratif et non limitatif, permettront de mieux comprendre l'invention.

### Exemple 1 : Préparation du N-cholestéryloxycarbonyl-D-glucamine

100 g (0,552 mole) de D-glucamine ont été mis en suspension, sous atmosphère inerte, dans 1 litre de N,N-diméthyl acétamide, puis 120 g (0,267 mole) de chloroformiate de cholestéryl ont été ajoutés par petites quantités. Le mélange réactionnel a été agité pendant 2 heures puis versé dans 10 litres d'eau légèrement acide. Le précipité formé a été séché et on a obtenu 112 g ( rendement 70 %) de poudre blanche.
Point de fusion : 126 °C
Le spectre RMN¹H est conforme à la structure attendue.

| Analyse élémentaire : C₃₄ H₅₉ N O₇ | | | | |
|---|---|---|---|---|
| | **%C** | **%H** | **%N** | **%O** |
| **Calculé** | 68,77 | 10,01 | 2,36 | 18,86 |
| **Trouvé** | 68,50 | 10,04 | 2,57 | 18,97 |

### Exemple 2 : Préparation du N-cholestéryloxycarbonyl-(1,1-dihydroxyméthyl)-éthylamine

Le composé a été synthétisé selon le mode opératoire de l'exemple 1, en utilisant:
- 18,5 g (0,0412 mole) de chloroformiate de cholestéryle
- 9,5 g (0,09 mole) de 2-amino-2-méthyl-1,3-propanediol
- 150 ml de N,N-diméthyl acétamide

On a obtenu 17,7 g ( rendement 83 %) de poudre blanche.
Point de fusion : 116 °C
Le spectre RMN¹H est conforme à la structure attendue.

| Analyse élémentaire : C₃₁ N₅₃ N O₄ | | | | |
|---|---|---|---|---|
| | **%C** | **%H** | **%N** | **%O** |
| **Calculé** | 74,23 | 10,71 | 2,71 | 12,36 |
| **Trouvé** | 74,28 | 10,64 | 2,80 | 12,42 |

### Exemple 3 : Etude des propriétés d'encapsulation des vésicules

A partir de lipides selon l'invention, on a préparé des vésicules à coeur aqueux dont la membrane comprend 90 % (poids/poids) de lipide selon l'invention et 10 % (poids/poids) de sel monosodique de glutamate de suif hydrogéné ("AMISOFT HS-11" de la société AJINOMOTO).

On a mesuré les capacités d'encapsulation des vésicules et le maintien de l'encapsulation dans les vésicules au cours du temps.
La capacité d'encapsulation a été déterminée par la mesure du taux de gonflement exprimé en microlitre/mg : il indique le nombre de microlitres de phase aqueuse que l'on peut encapsuler dans les vésicules en utilisant 1 mg de lipides.
Le maintien de l'encapsulation dans les vésicules au cours du temps a été déterminé par la mesure du taux de fuite à 15 jours et 30 jours : il indique le pourcentage de phase initialement encapsulée qui se retrouve à l'extérieur des vésicules.
Les méthodes de détermination du taux de gonflement et du taux de fuite sont bien connues de l'homme du métier.

L'étude a été réalisée pour deux lipides selon l'invention :
- lipide 1 : N-cholestéryloxycarbonyl-D-glucamine -D-glucamine (composé de l'exemple 1)
- lipide 2 : N-cholestéryloxycarbonyl-N-méthyl-D-glucamine (composé décrit dans Lange W., Amundson M., J. of Pharmaceutical Sciences, vol 51, n° 11, 1962).

On a obtenu les résultats suivants :

| | **Diamètre (nm)** | **Gonflement (µl/mg lipide)** | **Taux de fuite 15 jours** | **Taux de fuite 30 jours** |
|---|---|---|---|---|
| **Lipide 1** | 421 | 10 | 10 % | 10% |
| **Lipide 2** | 369 | 2,5 | 10 % | 10% |

On a constaté que les 2 lipides selon l'invention permettent de préparer des vésicules présentant une bonne stabilité dans le temps et une faible taux de fuite qui reste constant dans le temps. Par ailleurs, le lipide 1 présente de bonne aptitude à l'encapsulation qui est meilleure que celle du lipide 2.

### Exemple 4 : Etude de stabilité des vésicules

A partir des lipides 1 et 2 de l'exemple 3 (lipides selon l'invention), on a préparé des vésicules à coeur aqueux et on a étudié leur stabilité dans le temps.
On a ajouté un mélange de 90 % (poids/poids) de lipide de formule (I) selon l'invention et de 10 % de sel monosodique de glutamate de suif hydrogéné ("AMISOFT HS-11" de la société AJINOMOTO) dans un volume suffisant d'eau préalablement chauffé à 80°C. Après 30 minutes d'agitation, le mélange a été ramené à 60 °C puis homogénéisé à l'aide d'un homogénéisateur haute pression, par passage à 5 x 10⁷ Pa. On a ainsi obtenu des vésicules en dispersion dans l'eau.
On a mesuré les tailles des vésicules après leur formation (t=0) :
lipide 1 : 170 nm
lipide 2 : 182 nm
Puis on a conservé les dispersions obtenues pendant 2 mois à 4°C et à 45°C et on a mesuré à nouveau la taille des vésicules. On a constaté que la taille des vésicules n'avait pas changé pour les 2 lipides testés, et dans les deux conditions de température. Par ailleurs, on a constaté que la polydispersité des vésicules était constante et aucune recristallisation de produit n'est apparu.
Ces résultats obtenus montrent donc que les lipides selon l'invention présentent une bonne aptitude à former des vésicules stables.

### Exemple 5:

On a préparé un sérum hydratant pour le visage, ayant la composition suivante :
Phase A :
   - N-cholestéryloxycarbonyl-N-méthyl-D-glucamine (1) 3,6 g
   - sel monosodique de glutamate de suif hydrogéné vendu sous la dénomination AMISOFT HS-11 ®par AJINOMOTO 0,4 g
   - acétate de tocophérol 0,2 g
Phase B :
   - eau distillée 50 g
   - glycérine 5 g
Phase C :
   - mélange d'acide polycarboxyvinylique vendu sous la dénomination CARBOPOL 980® par la société GOODRICH 0,2 g
   - triéthanolamine 0,2 g
   - conservateurs qs
   - eau distillée qsp 100g
(1) ce composé est décrit dans Lange W., Amundson M., J. of Pharmaceutical Sciences, vol 51, n° 11, 1962.

La phase A a été ajoutée sous vive agitation dans la phase B préalablement chauffée à 80 °C. Après 30 minutes d'agitation, on a constaté au microscope la parfaite hydratation des lipides qui se présentent sous la forme de vésicules grossières.

Le mélange ainsi obtenu a été ramené à 60 °C puis homogénéisé à l'aide d'un homogénéisateur haute pression, par 3 passages à 5 x 10⁷ Pa . On a obtenu alors des vésicules ayant une taille moyenne de l'ordre de 200 nm.
La phase C a été ensuite ajoutée à température ambiante sous agitation à l'aide d'une défloculeuse (RAYNERI).
On a ainsi obtenu un sérum opalescent et fluide qui s'applique facilement sur le visage.

### Exemple 6:

On a préparé une crème de jour ayant la composition suivante :
Phase A :
   - N-cholestéryloxycarbonyl-D-glucamine (composé de l'exemple 1) 5,4 g
   - sel monosodique de glutamate de suif hydrogéné vendu sous la dénomination AMISOFT HS-11 ®par AJINOMOTO 0,6 g
Phase B :
   - eau distillée 40 g
   - glycérine 3 g
Phase C :
   - huile d'avocat 6 g
   - huile de silicone volatile 6 g
   - huile de paraffine 4 g
   - conservateurs 0,1 g
Phase D:
   - mélange d'acide polycarboxyvinylique vendu sous la dénomination CARBOPOL 980® par la société GOODRICH 0,4 g
   - triéthanolamine 0,4 g
   - eau distillée qsp 100g

La composition a été préparée selon un mode opératoire identique à celui de l'exemple 5.
La phase C a été dispersée dans le mélange des phases A et B puis homogénéisée. On a obtenu ainsi une dispersion d'huile stabilisée par les vésicules, la granulométrie de la dispersion d'huile étant de l'ordre de 250 nm. Enfin, la phase D a été ajoutée de la même façon que celle de l'exemple 5.
On a ainsi obtenu une crème de jour qui s'applique facilement sur le visage.

### Exemple 7 :

On a préparé une crème anti-solaire ayant la composition suivante :
Phase A :
   - N-cholestéryloxycarbonyl-D-glucamine 2,7 g
   - sel monosodique de glutamate de suif hydrogéné vendu sous la dénomination AMISOFT HS-11 ®par AJINOMOTO 0,3 g
   - acétate de tocophérol 0,3 g
Phase B :
   - eau distillée 40 g
   - glycérine 5 g
Phase C :
   - méthoxycinnamate d'octyle vendu sous la dénomination PARSOL MCX® par la société GIVAUDAN 15 g
   - huile de silicone volatile 6 g
Phase D:
   - mélange d'acide polycarboxyvinylique vendu sous la dénomination CARBOPOL 980® par la société GOODRICH 0,5 g
   - triéthanolamine 0,5 g
   - conservateurs qs
   - eau distillée qsp 100 g

### Exemple 8 :

On a préparé un lait parfumé ayant la composition suivante :
Phase A :
   - N-cholestéryloxycarbonyl-N-méthyl-D-glucamine 1,8 g
   - sel monosodique de glutamate de suif hydrogéné vendu sous la dénomination AMISOFT HS-11 ®par AJINOMOTO 0,2 g
Phase B :
   - eau distillée 15 g
   - glycérine 3 g
Phase C:
   - huile essentielle de bergamote 4 g
   - huile de silicone volatile 2 g
Phase D:
   - mélange d'acide polycarboxyvinylique vendu sous la dénomination CARBOPOL 980® par la société GOODRICH 0,2 g
   - triéthanolamine 0,2 g
   - conservateurs qs
   - eau distillée qsp 100 g

## Revendications

1. Composition comprenant une dispersion aqueuse de vésicules comprenant une membrane lipidique, **caractérisée par le fait que** ladite membrane lipidique comprend au moins un carbamate de formule (I) suivante : 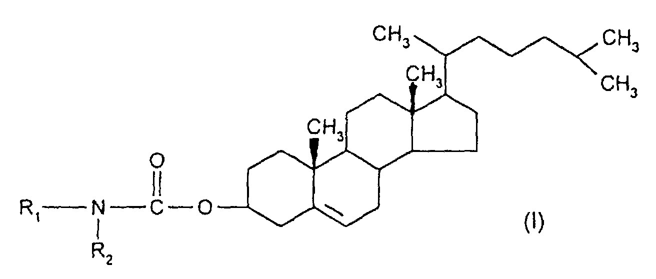 dans laquelle R₁ désigne un atome d'hydrogène ou un radical alkyle, linéaire ou ramifié, saturé ou insaturé, éventuellement hydroxylé, ayant de 1 à 6 atomes de carbone,
R₂ désigne un radical alkyle, linéaire ou ramifié, saturé ou insaturé, éventuellement hydroxylé, ayant de 3 à 6 atomes de carbone,
sous réserve que le groupement (R₁)(R₂)N- comporte au moins deux groupements hydroxyle.

2. Composition selon la revendication1, **caractérisée par le fait que** le composé de formule (I) est choisi parmi le N-cholestéryloxycarbonyl-N-méthyl-D-glucamine, le N-cholestéryloxycarbonyl-D-glucamine, le N-cholestéryloxycarbonyl-1,1-di(hydroxyméthyl)-éthylamine ou le N-cholestéryloxycarbonyl-2,3-dihydroxy-propyl-amine.

3. Composition selon l'une des revendications précédentes, **caractérisée par le fait qu'**elle comprend une dispersion de vésicules lipidiques à coeur aqueux.

4. Composition selon la revendication 3, **caractérisée par le fait que** la membrane lipidique des vésicules lipidiques à coeur aqueux comprend en outre au moins un lipide amphiphile ionique et éventuellement au moins un lipide amphiphile non ionique.

5. Composition selon la revendication 4, **caractérisée par le fait que** le rapport en poids entre la quantité de carbamate de formule (I) et la quantité de lipide amphiphile est compris entre 30/1 et 50/25 et/ou que le rapport en poids entre la quantité de membrane lipidique et la quantité de phase aqueuse de la dispersion est compris entre 1/1000 et 300/1000.

6. Composition selon l'une quelconque des revendications 3 à 5, **caractérisée par le fait que** la membrane lipidique des vésicules à coeur aqueux comprend en outre au moins un additif.

7. Composition selon la revendication 6, **caractérisé par le fait que** l'additif est choisi parmi les stérols, les alcools et diols à longue chaîne, les amines à longue chaîne et leurs dérivés ammonium quaternaire.

8. Composition selon la revendication 7, **caractérisée par le fait que** l'additif est le cholestérol.

9. Composition selon l'une des revendications 1 à 2, **caractérisée par le fait qu'**elle comprend une dispersion de vésicules lipidiques à coeur huileux.

10. Composition selon la revendication 9, **caractérisée par le fait que** les vésicules à coeur huileux sont des globules huileux en dispersion unitairement enrobés d'une couche monolamellaire ou oligolamellaire obtenue à partir d'au moins un carbamate de formule (I) et d'un tensioactif lipophile et/ou d'un tensioactif hydrophile.

11. Composition selon la revendication 10, **caractérisée par** le lait que la couche monolamellaire ou oligolamellaire comprend en outre soit un lipide amphiphile ionique, soit un acide gras associé à un agent basique dissous dans la phase aqueuse de la dispersion.

12. Composition selon l'une des revendications 4 et 11, **caractérisée par le fait que** les lipides amphiphiles ioniques sont choisis dans le groupe formé par les lipides anioniques neutralisés, les lipides amphotères et les dérivés alkylsulfoniques.

13. Composition selon la revendication 12, **caractérisée par le fait que** les lipides amphiphiles ioniques sont choisis dans le groupe formé par :
- les sels alcalins du dicétylphosphate, du dimyristylphosphate, du cholestérol-sulfate, du cholestérol-phosphate,
- les sels de lipoaminoacides,
- le sel de sodium de l'acide phosphatidique,
- les phospholipides,
- les dérivés alkylsulfoniques de formule : 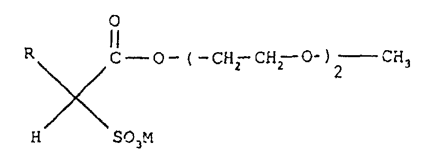 formule dans laquelle R représente des radicaux alkyles en C₁₂-C₂₂, en particulier les radicaux C₁₆H₃₃ et C₁₈H₃₇, pris en mélange ou séparément et M est un métal alcalin.

14. Composition selon la revendication 4, **caractérisée par le fait que** les lipides amphiphiles non ioniques sont choisis dans le groupe formé par :
(1) les éthers de polyglycérol, linéaires ou ramifiés, de formule (II) :
R¹⁰O-(-C₃H₅-(OH)O-]ₙ-H
dans laquelle :
. -C₃H₅(OH)O est représenté par les structures suivantes prises en mélange ou séparément :
-CH₂CHOHCH₂O ; 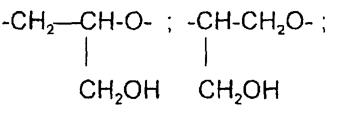
. n est une valeur statistique moyenne comprise entre 2 et 6 ;
. R¹⁰ représente :
(a) une chaîne aliphatique, linéaire ou ramifiée, contenant de 12 à 18 atomes de carbone ;
(b) un reste R¹¹CO, où R¹¹ est un radical aliphatique, linéaire ou ramifié, en C₁₁-C₁₇ ;
(c) un reste R¹²-[-OC₂H₃(R¹³)-]-, où :
. R¹² peut prendre la signification (a) ou (b) donnée pour R¹⁰ ;
. OC₂H₃(R¹³)- est représenté par les structures suivantes, prises en mélange ou séparément : 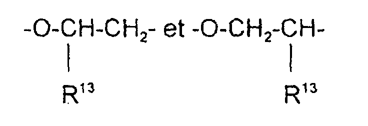 où R¹³ prend la signification (a) donnée pour R¹⁰ ;
(2) les alcools gras polyoxyéthylénés, les stérols polyoxyéthylénés ;
(3) les esters de polyols éventuellement polyoxyéthylénés ;
(4) les glycolipides naturels ou synthétiques ; et
(5) le stéarate de polyglycérol oxyéthyléné.

15. Composition selon l'une quelconque des revendications précédentes, **caractérisée par le fait que** les vésicules contiennent au moins un actif ayant une activité cosmétique et/ou dermopharmaceutique.

16. Composition selon la revendication 1 comprenant des vésicules lipidiques à coeur aqueux, lesdits vésicules comprenant une membrane lipidique obtenue à partir d'un mélange de composé de formule (I) et de sel monosodique de glutamate de suif hydrogéné.

17. Composition selon l'une quelconque des revendications précédentes, **caractérisée par le fait qu'**elle contient en outre au moins un liquide non miscible à l'eau, dispersé dans la phase aqueuse de la dispersion.

18. Composition selon la revendication 17, **caractérisée par le fait que** le liquide non miscible à l'eau est dispersé à l'aide des vésicules lipidiques.

19. Composition selon l'une quelconque des revendications précédentes, **caractérisée par le fait que** la phase aqueuse de la dispersion contient en outre un actif cosmétique et/ou dermopharmaceutique, et/ou un adjuvant choisi dans le groupe formé par les gélifiants, les conservateurs, les colorants, les opacifiants, les parfums, les pigments, les filtres solaires et les poudres à usage cosmétique.

20. Composition selon l'une des revendications précédentes, se présentant sous la forme d'une composition à usage topique, notamment une composition pour le soin et/ou le maquillage du visage, du corps et/ou du cuir chevelu.

21. Procédé de traitement non thérapeutique de la peau et/ou du cuir chevelu, **caractérisé par le fait qu'**on applique sur la peau ou le cuir chevelu, une composition selon l'une quelconque des revendications 1 à 20.

22. Composé de formule (I') suivante : 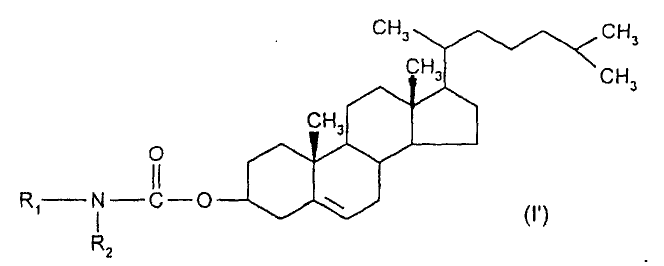 dans laquelle R₁ désigne un atome d'hydrogène ou un radical alkyle, linéaire ou ramifié, saturé ou insaturé, éventuellement hydroxylé, ayant de 1 à 6 atomes de carbone,
R₂ désigne un radical alkyle, linéaire ou ramifié, saturé ou insaturé, éventuellement hydroxylé, ayant de 3 à 6 atomes de carbone,
sous réserve que le groupement (R₁)(R₂)N- comporte au moins deux groupements hydroxylé,
à l'exception ;
- du N-cholestéryloxycarbonyl-N-méthyl-D-glucamine,
- des composés pour lesquels :
- soit R₁ désigne un atome d'hydrogène et R₂ est un groupement choisi parmi :
-CH(OH)-CH₂-CH₂-OH ; -CH₂-CH(OH)-CH₂-OH; -CH(CH₂OH)-CH₂-OH ; - CH₂-CH(OH)-CH₂-CH₂OH
- soit R₁ désigne un radical alkyle choisi dans le groupe formé par les radicaux méthyle, éthyle, n-propyl, isopropyl, n-butyl et t-butyl et R₂ désigne un groupement choisi dans le groupe formé par :
-CH₂-CH(OH)-CH₂-OH ; -CH(CH₂OH)-CH₂-OH
- soit R₁ et R₂ désignent un radical -CH₂-CH₂OH

23. Utilisation de vésicules comprenant une membrane lipidique qui comprend au moins un carbamate de formule (I), pour disperser ou aider à la mise en dispersion, d'un liquide non miscible à l'eau, dans une phase aqueuse.

24. Utilisation de carbamate de formule (I) pour la préparation de vésicules lipidiques.

## Patentansprüche

1. Zusammensetzung, die eine wässerige Dispersion von Lipidvesikeln enthält, die eine Lipidmembran aufweisen, **dadurch gekennzeichnet, daß** die Lipidmembran mindestens eine Carbamat der folgenden Formel (I) enthält: 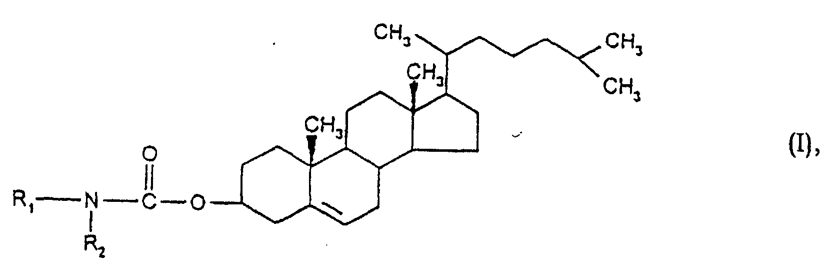 worin bedeuten:
- R₁ ein Wasserstoffatom oder eine geradkettige oder verzweigte, gesättigte oder ungesättigte, gegebenenfalls hydroxylierte Alkylgruppe mit 1 bis 6 Kohlenstoffatomen;
- R₂ eine geradkettige oder verzweigte, gesättigte oder ungesättigte, gegebenenfalls hydroxylierte Alkylgruppe mit 3 bis 6 Kohlenstoffatomen;
mit der Maßgabe, daß die Gruppe (R₁)(R₂)N- mindestens zwei Hydroxygruppen aufweist.

2. Zusammensetzung nach Anspruch 1, **dadurch gekennzeichnet, daß** die Verbindung der Formel (I) unter N-Cholesteryloxycarbonyl-N-methyl-D-glucamin, N-Cholesteryloxycarbonyl-D-giucamin, N-Cholesteryloxycarbonyl-1,1-di(hydroxymethyl)-ethylamin oder N-Cholesteryloxycarbonyl-2,3-dihydroxypropylamin ausgewählt ist.

3. Zusammensetzung nach einem der vorhergehenden Ansprüche, **dadurch gekennzeichnet, daß** sie eine Dispersion von Lipidvesikeln mit wässerigem inneren Kompartiment enthält.

4. Zusammensetzung nach Anspruch 3, **dadurch gekennzeichnet, daß** die Lipidmembran der Lipidvesikel mit wässerigem inneren Kompartiment ferner mindestens ein ionisches amphiphiles Lipid und gegebenenfalls mindestens ein nichtionisches amphiphiles Lipid enthält.

5. Zusammensetzung nach Anspruch 4, **dadurch gekennzeichnet, daß** das Gewichtsverhältnis der Menge des Carbamats der Formel (I) und der Menge des amphiphilen Lipids im Bereich von 30/1 bis 50/25 und/oder das Gewichtsverhältnis des Mengenanteils der Lipidmembran und der Menge der wässerigen Phase der Dispersion im Bereich von 1/1000 bis 300/1000 liegt.

6. Zusammensetzung nach einem der Ansprüche 1 bis 3, **dadurch gekennzeichnet, daß** die Lipidmembran der Vesikel mit wässerigem inneren Kompartiment ferner mindestens einen Zusatzstoff enthält.

7. Zusammensetzung nach Anspruch 6, **dadurch gekennzeichnet, daß** der Zusatzstoff unter den Sterinen, Alkoholen und Diolen mit langen Ketten, Aminen mit langen Ketten und deren quartären Ammoniumderivaten ausgewählt ist.

8. Zusammensetzung nach Anspruch 7, **dadurch gekennzeichnet, daß** der Zusatzstoff das Cholesterin ist.

9. Zusammensetzung nach einem der Ansprüche 1 bis 2, **dadurch gekennzeichnet, daß** sie eine Dispersion von Lipidvesikeln mit öligem inneren Kompartiment enthält.

10. Zusammensetzung nach Anspruch 9, **dadurch gekennzeichnet, daß** die Lipidvesikel mit öligem inneren Kompartiment Ölkügelchen in Dispersion sind, die von einer monolamellaren oder oligolamellaren Schicht umhüllt sind, die aus mindestens einem Carbamat der Formel (I) und einem lipophilen grenzflächenaktiven Stoff und/oder einem hydrophilen grenzflächenaktiven Stoff hergestellt ist.

11. Zusammensetzung nach Anspruch 10, **dadurch gekennzeichnet, daß** die monolamellare oder oligolamellare Schicht ferner entweder ein ionisches amphiphiles Lipid oder eine Fettsäure in Kombination mit einem basischen Mittel enthält, welche in der wässerigen Phase der Dispersion gelöst sind.

12. Zusammensetzung nach einem der Ansprüche 4 bis 11, **dadurch gekennzeichnet, daß** die amphiphilen ionischen Lipide unter den neutralisierten anionischen Lipiden, den amphoteren Lipiden und den Alkylsulfonsäurederivaten ausgewählt sind.

13. Zusammensetzung nach Anspruch 12, **dadurch gekennzeichnet, daß** die amphiphilen ionischen Lipide ausgewählt sind unter:
- den Alkalisalzen von Dicetyl- und Dimyristylphosphat, von Cholesterinsulfat und von Cholesterinphosphat;
- den Salzen von Lipoaminosäuren;
- dem Natriumsalz von Phosphatidsäure;
- Phospholipiden;
- Alkylsulfonsäurederivaten der Formel: 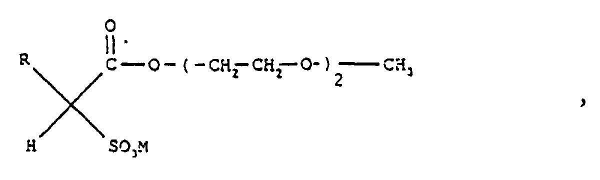 worin R C₁₂₋₂₂-Alkylgruppen und insbesondere die Gruppen C₁₆H₃₃ und C₁₈H₃₇ bedeutet, wobei diese Gruppen im Gemisch oder einzeln vorhanden sein können, und M ein Alkalimetall ist.

14. Zusammensetzung nach Anspruch 4, **dadurch gekennzeichnet, daß** die amphiphilen nichtionischen Lipide ausgewählt sind unter:
1) geradkettigen oder verzweigten Polyglycerinethern der Formel (II):
R¹⁰O-[-C₃H₅-(OH)O-]ₙ-H,
worin bedeuten:
· -C₃H₅(OH)O- die folgenden Strukturen, die im Gemisch oder einzeln vorliegen können:
-CH₂CHOHCH₂O-; 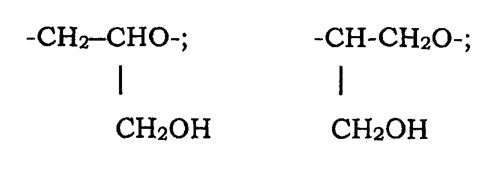
· n einen statistischen Mittelwert im Bereich von 2 bis 6;
· R¹⁰;
(a) eine geradkettige oder verzweigte aliphatische Kette mit 12 bis 18 Kohlenstoffatomen;
(b) eine Gruppe R¹¹CO, wobei R¹¹ eine geradkettige oder verzweigte aliphatische Gruppe mit 11 bis 17 Kohlenstoffatomen bedeutet;
(c) eine Gruppe R¹²-[-OC₂H₃(R¹³)-]-, worin:
· R¹² die für R¹⁰ angegebenen Bedeutungen (a) oder (b) haben kann;
· OC₂H₃(R¹³) den folgenden Strukturen entspricht, wobei sie im Gemisch oder einzeln vorliegen können: 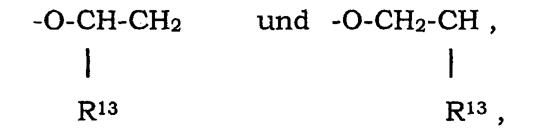 wobei R¹³ die für R¹⁰ unter (a) angegebene Bedeutung hat;
2) polyethoxylierten Fettalkoholen und polyethoxylierten Sterinen;
3) Estern von Polyolen, die gegebenenfalls polyethoxyliert sind;
4) natürlichen oder synthetischen Glykolipiden; und
5) ethoxyliertem Polyglycerinstearat.

15. Zusammensetzung nach einem der vorhergehenden Ansprüche, **dadurch gekennzeichnet, daß** die Vesikel mindestens einen Wirkstoff mit kosmetischer und/oder dermopharmazeutischer Aktivität enthalten.

16. Zusammensetzung nach Anspruch 1, die Lipidvesikel mit wässerigem inneren Kompartiment enthält, wobei die Vesikel eine Lipidmembran aufweisen, die ausgehend von einem Gemisch einer Verbindung der Formel (I) und dem Mononatriumsalz von hydriertem Talgglutamat hergestellt wird.

17. Zusammensetzung nach einem der vorhergehenden Ansprüche, **dadurch gekennzeichnet, daß** sie ferner mindestens eine nicht mit Wasser mischbare Flüssigkeit enthält, die in der wässerigen Phase der Dispersion dispergiert ist.

18. Zusammensetzung nach Anspruch 17, **dadurch gekennzeichnet, daß** die nicht mit Wasser mischbare Flüssigkeit mit Hilfe von Lipidvesikeln dispergiert ist.

19. Zusammensetzung nach einem der vorhergehenden Ansprüche, **dadurch gekennzeichnet, daß** die wässerige Phase der Dispersion außerdem einen kosmetischen und/oder dermopharmazeutischen Wirkstoff und/oder einen Hilfsstoff enthält, das unter den Gelbildnern, Konservierungsmitteln, Farbmitteln, Trübungsmitteln, Parfums, Pigmenten, Sonnenschutzfiltern und Pulvern zur kosmetischen Verwendung ausgewählt ist.

20. Zusammensetzung nach einem der vorhergehenden Ansprüche, die in Form einer Zusammensetzung zur topischen Anwendung und insbesondere als Zusammensetzung zur Pflege und/oder zum Schminken des Gesichts, des Körpers und/oder der Kopfhaut vorliegt.

21. Verfahren zur nicht therapeutischen Behandlung der Haut und/oder der Kopfhaut, **dadurch gekennzeichnet, daß** auf die Haut oder die Kopfhaut eine Zusammensetzung nach einem der Ansprüche 1 bis 20 aufgebracht wird.

22. Verbindungen der folgenden Formel (I'): 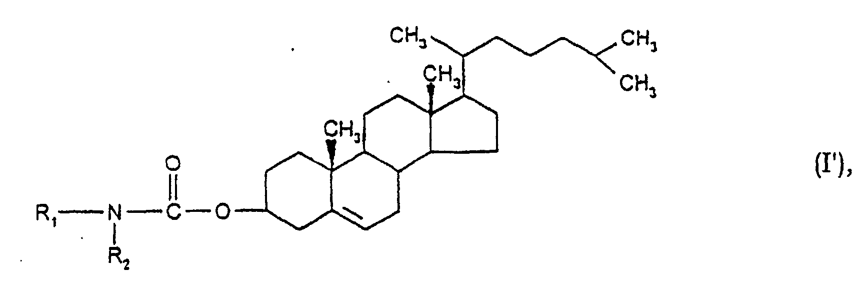 worin bedeuten:
- R₁ ein Wasserstoffatom oder eine geradkettige oder verzweigte, gesättigte oder ungesättigte, gegebenenfalls hydroxylierte Alkylgruppe mit 1 bis 6 Kohlenstoffatomen;
- R₂ eine geradkettige oder verzweigte, gesättigte oder ungesättigte, gegebenenfalls hydroxylierte Alkylgruppe mit 3 bis 6 Kohlenstoffatomen;
mit der Maßgabe, daß die Gruppe (R₁)(R₂)N- mindestens zwei Hydroxygruppen aufweist,
mit Ausnahme der folgenden Verbindungen:
- N-Cholesteryloxycarbonyl-N-methyl-D-glucamin,
- Verbindungen, worin bedeuten:
· R₁ ein Wasserstoffatom und R₂ eine Gruppe, die unter:
-CH(OH)-CH₂-CH₂-OH; -CH₂-CH(OH)-CH₂-OH; -CH(CH₂OH)-CH₂-OH; -CH₂-CH(OH)-CH₂-CH₂-OH ausgewählt ist; oder
· R₁ eine Alkylgruppe, die unter den Gruppen Methyl, Ethyl, n-Propyl, Isopropyl und t-Butyl ausgewählt ist, und R₂ eine Gruppe, die unter:
- CH₂-CH(OH)-CH₂-OH und -CH(CH₂OH)-CH₂-OH ausgewählt ist;
oder
· R₁ und R₂ die Gruppe -CH₂-CH₂-OH.

23. Verwendung von Vesikeln, die eine Lipidmembran aufweisen, die mindestens ein Carbamat der Formel (I) enthält, um eine nicht mit Wasser mischbare Flüssigkeit in einer wässerigen Phase zu dispergieren oder zu ihrer Dispersion beizutragen.

24. Verwendung eines Carbamats der Formel (I) zur Herstellung von Lipidvesikeln.

## Claims

1. Composition comprising an aqueous dispersion of vesicles comprising a lipid membrane, **characterized in that** the said lipid membrane comprises at least one carbamate of following formula (I): 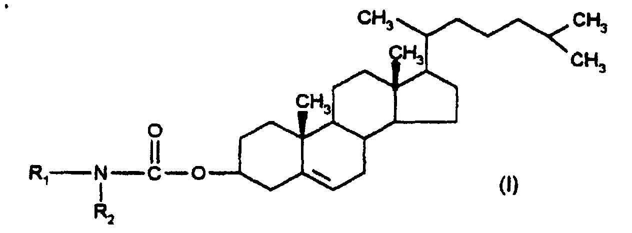 in which formula R₁ denotes a hydrogen atom or an optionally hydroxylated, saturated or unsaturated, linear or branched alkyl radical having from 1 to 6 carbon atoms,
R₂ denotes an optionally hydroxylated, saturated or unsaturated, linear or branched alkyl radical having from 3 to 6 carbon atoms,
with the proviso that the (R₁) (R₂)N- group comprises at least two hydroxyl groups.

2. Composition according to Claim 1, **characterized in that** the compound of formula (I) is chosen from N-chlolesteryloxycarbonyl-N-methyl-D-glucamine, N-cholesteryloxycarbonyl-D-glucamine, N-cholesteryloxycarbonyl-1,1-di(hydroxymethyl)ethylamine or N-cholesteryloxycarbonyl-2,3-dihydroxypropylamine.

3. Composition according to either of the preceding claims, **characterized in that** it comprises a dispersion of lipid vesicles with an aqueous core.

4. Composition according to Claim 3, **characterized in that** the lipid membrane of the lipid vesicles with an aqueous core additionally comprises at least one ionic amphiphilic lipid and optionally at least one non-ionic amphiphilic lipid.

5. Composition according to Claim 4, **characterized in that** the ratio by weight of the amount of carbamate of formula (I) to the amount of amphiphilic lipid is between 30/1 and 50/25 and/or that the ratio by weight of the amount of lipid membrane to the amount of aqueous phase of the dispersion is between 1/1000 and 300/1000.

6. Composition according to any one of Claims 3 to 5, **characterized in that** the lipid membrane of the vesicles with an aqueous core additionally comprises at least one additive.

7. Composition according to Claim 6, **characterized in that** the additive is chosen from sterols, long-chain alcohols and diols, or long-chain amines and their quaternary ammonium derivatives.

8. Composition according to Claim 7, **characterized in that** the additive is cholesterol.

9. Composition according to either of Claims 1 and 2, **characterized in that** it comprises a dispersion of lipid vesicles with an oily core.

10. Composition according to Claim 9, **characterized in that** the vesicles with an oily core are dispersed oily globules individually coated with a monolamellar or oligolamellar layer obtained from at least one carbamate of formula (I) and from a lipophilic surfactant and/or from a hydrophilic surfactant.

11. Composition according to Claim 10, **characterized in that** the monolamellar or oligolamellar layer additionally comprises either an ionic amphiphilic lipid or a fatty acid, in combination with a basic agent dissolved in the aqueous phase of the dispersion.

12. Composition according to either of Claims 4 and 11, **characterized in that** the ionic amphiphilic lipids are chosen from the group formed by neutralized anionic lipids, amphoteric lipids and alkylsulphonic derivatives.

13. Composition according to Claim 12, **characterized in that** the ionic amphiphilic lipids are chosen from the group formed by:
- alkaline salts of dicetyl phosphate, of dimyristyl phosphate, of cholesterol sulphate or of cholesterol phosphate,
- salts of lipoamino acids,
- the sodium salt of phosphatidic acid,
- phospholipids,
- alkylsulphonic derivatives of formula: 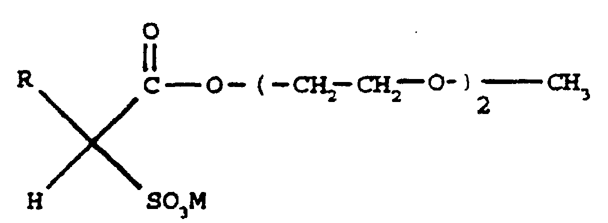 in which formula R represents C₁₂-C₂₂ alkyl radicals, in particular the C₁₆H₃₃ and C₁₈H₃₇ radicals, taken as a mixture or separately, and M is an alkali metal.

14. Composition according to Claim 4, **characterized in that** the non-ionic amphiphilic lipids are chosen from the group formed by:
(1) linear or branched ethers of polyglycerol of formula (II)
R¹⁰O-[-C₃H₅-(OH)O-]ₙ-H
in which:
• -C₃H₅(OH)O is represented by the following structures, taken as a mixture or separately:
-CH₂CHOHCH₂O- ; 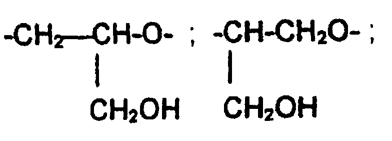
• n is a mean statistical value of between 2 and 6;
• R¹⁰ represents:
(a) a linear or branched aliphatic chain comprising from 12 to 18 carbon atoms;
(b) an R¹¹CO residue, where R¹¹ is a linear or branched C₁₁-C₁₇ aliphatic radical;
(c) an R¹²-[-OC₂H₃(R¹³)-)- residue, where:
• R¹² can take the meaning (a) or (b) given for R¹⁰;
• OC₂H₃(R¹³)- is represented by the following structures, taken as a mixture or separately: 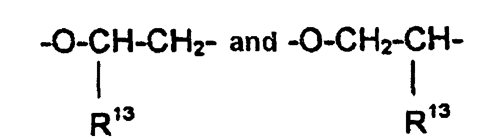 where R¹³ takes the meaning (a) given for R¹⁰;
(2) polyoxyethylenated fatty alcohols or polyoxyethylenated sterols;
(3) optionally polyoxyethylenated polyol esters;
(4) natural or synthetic glycolipids; and
(5) oxyethylenated polyglycerol stearate.

15. Composition according to any one of the preceding claims, **characterized in that** the vesicles comprise at least one active compound having a cosmetic and/or dermopharmaceutical activity.

16. Composition according to Claim 1 comprising lipid vesicles with an aqueous core, the said vesicles comprising a lipid membrane obtained from a mixture of compound of formula (I) and of monosodium salt of hydrogenated tallow glutamate.

17. Composition according to any one of the preceding claims, **characterized in that** it additionally contains at least one water-immiscible liquid dispersed in the aqueous phase of the dispersion.

18. Composition according to Claim 17, **characterized in that** the water-immiscible liquid is dispersed using the lipid vesicles.

19. Composition according to any one of the preceding claims, **characterized in that** the aqueous phase of the dispersion additionally comprises a cosmetic and/or dermopharmaceutical active compound and/or an adjuvant chosen from the group formed by gelling agents, preservatives, colorants, opacifiers, fragrances, pigments, sunscreens and powders for cosmetic use.

20. Composition according to one of the preceding claims, which is provided in the form of a composition for topical use, in particular a composition for caring for and/or making up the face, body and/or scalp.

21. Process for the non-therapeutic treatment of the skin and/or of the scalp, **characterized in that** a composition according to any one of Claims 1 to 20 is applied to the skin or the scalp.

22. Compound of following formula (I'): 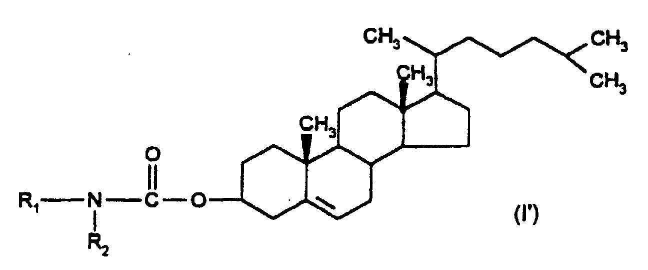 in which formula R₁ denotes a hydrogen atom or an optionally hydroxylated, saturated or unsaturated, linear or branched alkyl radical having from 1 to 6 carbon atoms,
R₂ denotes an optionally hydroxylated, saturated or unsaturated, linear or branched alkyl radical having from 3 to 6 carbon atoms,
with the proviso that the (R₁)(R₂)N- group comprises at least two hydroxyl groups,
with the exception:
- of N-cholesteryloxycarbonyl-N-methyl-D-glucamine,
- of the compounds in which:
- either R₁ denotes a hydrogen atom and R₂ is a group chosen from:
-CH(OH)-CH₂-CH₂-OH; -CH₂-CH(OH)-CH₂-OH; -CH(CH₂OH)-CH₂-OH; -CH₂-CH(OH)-CH₂-CH₂OH
- or R₁ denotes an alkyl radical chosen from the group formed by the methyl, ethyl, n-propyl, isopropyl, n-butyl and t-butyl radicals and R₂ denotes a group chosen from the group formed by:
-CH₂-CH(OH)-CH₂-OH; -CH(CH₂OH)-CH₂-OH
- or R₁ and R₂ denote a -CH₂-CH₂OH radical.

23. Use of vesicles comprising a lipid membrane which comprises at least one carbamate of formula (I) for dispersing or helping in the dispersion of a water-immiscible liquid in an aqueous phase.

24. Use of carbamate of formula (I) for the preparation of lipid vesicles.
